(19) Europäisches Patentamt / European Patent Office / Office européen des brevets

(11) **EP 3 865 103 B1**

(12) **EUROPEAN PATENT SPECIFICATION**

(45) Date of publication and mention
of the grant of the patent:
**29.01.2025 Bulletin 2025/05**

(21) Application number: **19935749.2**

(22) Date of filing: **04.12.2019**

(51) International Patent Classification (IPC):
*A61F 13/49* ^(2006.01)        *A61F 13/514* ^(2006.01)

(52) Cooperative Patent Classification (CPC):
**A61F 13/49012; A61F 13/4902; A61F 13/51464;
A61F 13/51478;** A61F 2013/49023

(86) International application number:
**PCT/JP2019/047521**

(87) International publication number:
**WO 2021/111570 (10.06.2021 Gazette 2021/23)**

(54) **ABSORBENT ARTICLE**

ABSORBIERENDER ARTIKEL

ARTICLE ABSORBANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**18.08.2021 Bulletin 2021/33**

(73) Proprietor: **KAO CORPORATION
Tokyo 103-8210 (JP)**

(72) Inventors:
• **ISHIBASHI, Kyoko
Tochigi 3213497 (JP)**
• **FUKUDA, Yuko
Tochigi 3213497 (JP)**

(74) Representative: **Vossius & Partner
Patentanwälte Rechtsanwälte mbB
Siebertstrasse 3
81675 München (DE)**

(56) References cited:
WO-A1-2018/235211    JP-A- 2009 195 647
JP-A- 2009 195 647    JP-A- 2017 121 387
JP-A- 2017 121 387    JP-A- 2019 115 524
JP-A- 2019 115 524    US-B2- 9 011 404

**Description**

Technical Field

The present invention relates to an absorbent article.

Background Art

Absorbent articles having an outer cover laminate comprising at least two sheets and elastic members therebetween are known. For instance, the assignee common to this application has proposed an absorbent article including an outer cover having a laterally stretchable elasticized region, in which the sheets composing the outer cover are bonded to each other at bonds spaced in both the lateral direction and a direction perpendicular to the lateral direction (Patent Literatures 1 and 2 listed below). According to Patent Literature 1, the elasticized region providing waist gathers forms higher folds or winkles than the elasticized region providing below-waist gathers. The absorbent article of Patent Literature 2 has bonds with different bonding strengths.

Citation List

Patent Literature:

Patent Literature 1: JP 2008-173286A
Patent Literature 2: JP 2017-121387A

Summary of Invention

The present invention provides an absorbent article including an absorbent assembly having an absorbent member and an outer cover disposed on the non-skin facing side of the absorbent assembly. The absorbent article has a front section adapted to be worn about the front of a wearer, a rear section adapted to be worn about the back of the wearer, and a crotch section located between the front and rear sections. The absorbent article has a longitudinal direction corresponding to the direction from the front section to the rear section through the crotch section and a lateral direction perpendicular to the longitudinal direction. The outer cover includes an outer sheet on the non-skin facing side defining the exterior side of the absorbent article, an inner sheet on the skin facing side of the outer sheet, and a plurality of elastic members between the outer and inner sheets.

The outer cover preferably has a waist elasticized region located longitudinally distal to the absorbent assembly and a lower elasticized region longitudinally proximal to the waist elasticized region and longitudinally overlapping the absorbent assembly.

The outer and inner sheets of the outer cover are preferably bonded at bonds spaced apart in the lateral direction.

The outer cover preferably has, between laterally adjacent bonds, a non-bonded region where the outer and the inner sheets are not bonded to each other continuously in the longitudinal direction.

The outer sheet is preferably configured to bulge away from the skin of a wearer on contraction of the elastic members to form a plurality of longitudinally extending folds.

The waist elasticized region preferably has a lower bond strength than the lower elasticized region.

Brief Description of Drawings

[Fig. 1] Fig. 1 is a perspective of a disposable pull-on diaper according an embodiment of the present invention.
[Fig. 2] Fig. 2 is a plan of the diaper of Fig. 1 in its flat-out, uncontracted state.
[Fig. 3] Fig. 3 is a perspective of an elasticized region shown in Fig. 1.
[Fig. 4] Fig. 4a is an enlarged plan of the elasticized region in Fig. 1 illustrating the arrangement of bonds before a wearer moves, and Fig. 4b is a cross-section of the waist elasticized region before a wearer moves, taken in the lateral direction.
[Fig. 5] Fig. 5a is an enlarged plan of the elasticized region in Fig. 1, illustrating the arrangement of bonds after a wearer moves, and Fig. 5b is a cross-section of the waist elasticized region after the wearer moves, taken along the lateral direction.

**EP 3 865 103 B1**

[Fig. 6] Fig. 6a and Fig. 6b are each an enlarged plan of the waist and lower elasticized regions in which the bonds are arranged in another arrangement.

[Fig. 7] Fig. 7 is equivalent to Figs. 6, in which the bonds in the waist and the lower elasticized regions are arranged in still another arrangement.

[Fig. 8] Fig. 8 is equivalent to Figs. 6, in which the bonds in the waist and the lower elasticized regions are arranged in yet another arrangement.

Detailed Description of the Invention

The absorbent member of ordinary absorbent articles is a main fluid absorbing element that absorbs body fluids, such as urine. Upon fluid absorption, the absorbent member increases in weight and, as a result, can sag to cause a body fluid such as urine to leak. On the other hand, the outer cover, which comes into contact with the skin of a wearer while worn, is required to be conformable to the body contour so as to reduce chafing of the wearer's skin. The absorbent articles disclosed in Patent Literatures 1 and 2 leave room for improvements on both prevention of the diaper from sagging after fluid absorption and conformability of the outer cover to the wearer's body.

The present invention relates to an absorbent article configured to be effectively prevented from sagging after fluid absorption and exhibiting excellent conformability to the wearer's body.

The absorbent article of the present invention will be described on the basis of its preferred embodiments with reference to the accompanying drawings.

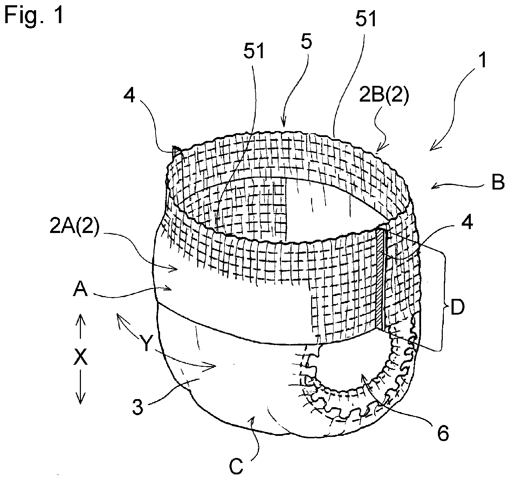

A diaper 1 according to one embodiment of the invention (hereinafter "the diaper 1") will be elaborated with reference to Figs. 1 through 5. The diaper 1 is of pull-on type as illustrated in Fig. 1 and includes an absorbent assembly 3 having an absorbent member 33 and an outer cover 2 disposed on the non-skin facing side of the absorbent assembly 3. The diaper 1 has a front section A adapted to be worn about the front of a wearer while worn and a rear section B adapted to be worn about the back of a wearer while worn. The front section A and the rear section B have their lateral side edges joined together to form a pair of side seams 4 and 4, thereby to form a waist opening 5 and a pair of leg openings 6. The diaper 1 also has a crotch section C intermediate between the front section A and the rear section B and adapted to be worn about the crotch of a wearer.

As used herein, the term "skin facing side" refers to the side of an absorbent article or a member constituting the absorbent article (e.g., the absorbent member 33) that faces the wearer's skin while worn. The term "non-skin facing side" refers to the side of an absorbent article or a member constituting the absorbent article that faces away from the wearer's skin while worn. That is, the skin facing side is relatively closer to the wearer's skin, while the non-skin facing side is relatively farther from the wearer's skin. The expression "while worn" means the state of an absorbent article applied in the right position to the body of a wearer.

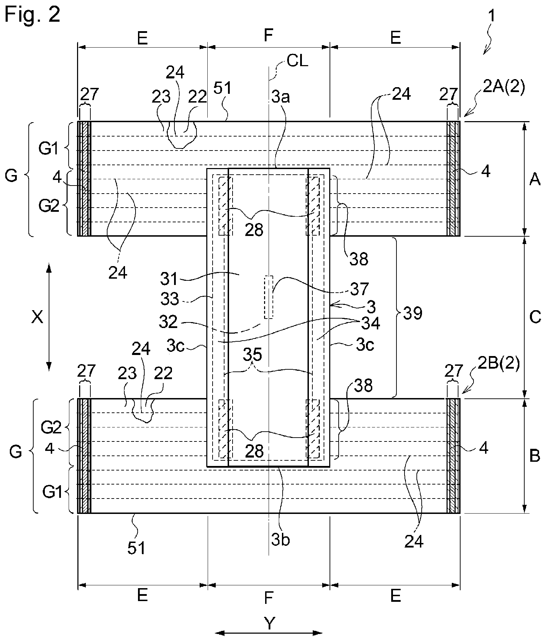

The diaper 1 has a longitudinal direction X corresponding to the direction from the front section A to the rear section B through the crotch section C and a lateral direction Y perpendicular to the longitudinal direction X. The longitudinal direction X is usually coincident with the longitudinal direction of the absorbent assembly 3. As illustrated in Fig. 2, the lateral direction Y is perpendicular to the longitudinal direction X in a flat-out, uncontracted state of the diaper. The front section A and the rear section B joined together form a tubular, lower torso portion D (Fig. 1), and the lateral direction Y is coincident with the circumferential direction of the lower torso portion D. The phrase "flat-out, uncontracted state" means a state in which the diaper 1 is opened by tearing the side seams 4 apart and with every elastic member stretched out until the diaper is straightened up to its design dimension, i.e., the dimension of the diaper in a flat-out configuration with any influences of elastic members eliminated.

Since the longitudinal direction X corresponds to the vertical direction of the front section A and the rear section B while worn, a location closer to, or a direction toward, the waist opening 5 in the front section A or the rear section B may be expressed by the word "upper" or "upward", and an opposite location or direction toward, the crotch section C may also be expressed by the word "lower" or "downward".

In the present embodiment, the outer cover 2 of the diaper 1 has a front panel 2A defining the front section A and a rear panel 2B defining the rear section B. The front panel 2A and the rear panel 3B are joined to each other at the pair of side seams 4 and 4. As illustrated in Fig. 2, the absorbent assembly 3 fixedly bridges the lateral middle portion F of the front panel 2A and the lateral middle portion F (hereinafter "the middle portion F") of the rear panel 2B in an overlapping relation. The absorbent assembly 3 is bonded to the front panel 2A and the rear panel 2B over the entire area or in part of the respective overlaps by a known bonding means, such as adhesive bonding. The outer cover 2 has, along the lateral direction Y, the middle portion F that overlaps the absorbent assembly 3 and a side portion E or E on either side of the middle portion F (see Fig. 2).

Each side seam 4 may have an unbonded region where the front panel 2A and the rear panel 2B are not bonded along the lateral side edge with a small width, e.g., 10 mm or less. In that case, too, the front panel 2A and the rear panel 2B are said to be joined together along the lateral side edges.

As illustrated in Fig. 2, the diaper 1 of the embodiment is bilaterally symmetric about the longitudinal centerline CL extending in the longitudinal direction X. The description of the diaper 1 will generally be confined to the right-hand side of Fig. 2, it being understood that the same applies to the left-hand side except that left and right are reversed.

The front and rear panel 2A and 2B of the diaper 1 of the embodiment each include an outer sheet 22 on the non-skin facing side that defines the exterior of the diaper 1, an inner sheet 23 laid on the skin facing side of the outer sheet 22, and a plurality of elastic members 24 disposed in their stretched state between the two sheets 22 and 23 as shown in Fig. 2.

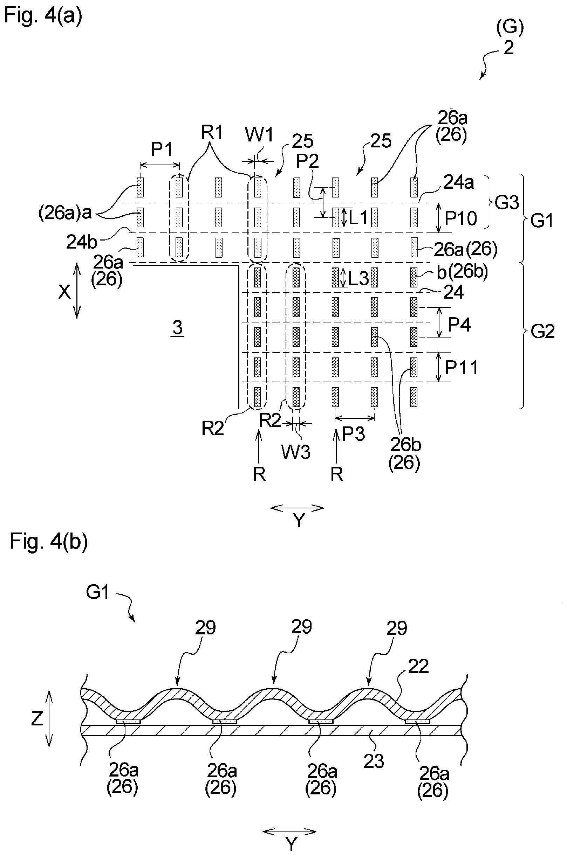

The outer cover 2, i.e., the combination of the front panel 2A and the rear panel 2B, has an elasticized region G that extends and contracts in the lateral direction Y with the extension and contraction of the elastic members 24 as shown in Fig. 2. The elasticized region G includes a waist elasticized region G1 longitudinally distal to the absorbent assembly 3 and a lower elasticized region G2 longitudinally proximal to the waist elasticized region G1. The lower elasticized region G2 is located in a region longitudinally overlapping the absorbent assembly 3. The waist elasticized region G1 includes a waist edge elasticized region G3 close to the waist opening 5 as illustrated in Fig. 4a.

The waist elasticized region G1 is formed outward of each of the longitudinal ends 3a and 3b of the absorbent assembly 3, having no longitudinal overlap with the absorbent assembly 3. The waist elasticized region G1 is a region adapted to be worn about the waist of a wearer. The waist edge elasticized region G3 is a subregion of the waist elasticized region G1 and is located immediately along the edge of the waist opening 5.

The plurality of elastic members 24 in the embodiment that are longitudinally spaced from each other will be designated a first elastic member 24a, a second elastic member 24b, a third elastic member 24c, ⋯ in order from the waist opening 5 toward the crotch section C. The first elastic member 24a is the nearest to the waist opening 5. The waist edge elasticized region G3 corresponds to the region from the waist opening 5 to the second elastic member 24b.

The lower elasticized region G2 corresponds to the region from the longitudinal end 3a or 3b of the absorbent assembly 3 to the lower (proximal) end of each side seal 4.

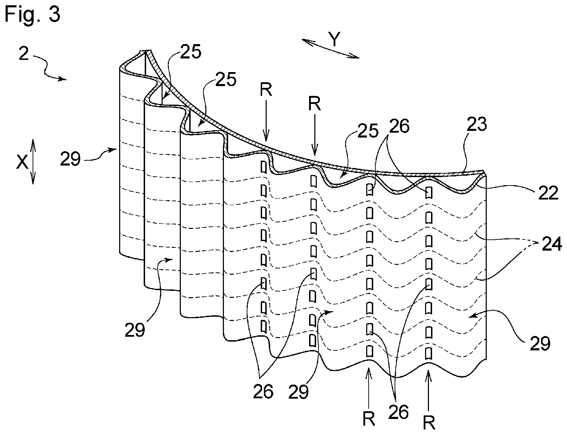

The outer sheet 22 and the inner sheet 23 are joined at a plurality of bonds 26 laterally spaced in the elasticized region G of the outer cover 2. In the embodiment, as illustrated in Fig. 3, the bonds 26 are discrete from one another, i.e., spaced from one another in both the lateral direction Y and the longitudinal direction X. In the embodiment, the bonds 26, at which the outer sheet 22 and the inner sheet 23 are bonded, line up at a spacing in the longitudinal direction X to make a plurality of columns R of bonds in the elasticized region G of each of the front panel 2A and the rear panel 2B, and the plurality of columns R of the bonds 26 are arranged at a spacing in the lateral direction Y (see Fig. 3).

As illustrated in Fig. 2, the front panel 2A and the rear panel 2B each have a pair of distal fixed regions 27 and 27 one on either side of the longitudinal centerline CL, where the outer and inner sheets 22 and 23 are adhesively bonded to each other. The front panel 2A and the rear panel 2B each also have a proximal fixed region 28 near each lateral side edge 3c of the absorbent assembly 3, where the outer and inner sheets 22 and 23 are adhesively bonded to each other. The pair of distal fixed regions 27 and 27 is spaced away laterally outward from the proximal fixed region 28 in each of the front panel 2A (front section A) and the rear panel 2B (rear section B). Specifically, the pair of distal fixed regions 27 and is formed along each of the lateral side edges of the front section A and the rear section B. It is preferred that the distal fixed region 27 overlap the side seam 4 partly or entirely. The adhesive in the distal fixed region 27 is preferably present discontinuously in the longitudinal direction X. For instance, it is more preferred for the feel to the touch of the outer cover 2 and the handle of the lateral side edges of the outer cover 2 that an adhesive for fixing the elastic members 24 to the sheets be present only at the parts where the elastic members 24 are disposed.

The waist elasticized region G1 has a plurality of elastic members 24 spanning between the pair of distal fixed regions 27 and 27. These elastic members 24 are individually fixed to the two sheets 22 and 23 at the opposed distal fixed region 27 but not fixed to either the sheet 22 or 23 in the region between the pair of distal fixed regions 27 and 27.

The lower elasticized region G2 has a plurality of elastic members 24 spanning between the distal fixed region 27 and the proximal fixed region 28. These elastic members 24 are individually fixed to the two sheets 22 and 23 constituting the outer cover 2 at each of the distal fixed region 27 and the proximal fixed region 28 but not fixed to either the sheet 22 or 23 in the region between the distal fixed region 27 and the proximal fixed region 28.

The proximal fixed region 28 may be located to entirely overlap the absorbent assembly 3 as illustrated in Fig. 2, or may be located to straddle the inside and outside of the side edge 3c of the absorbent assembly 3. Otherwise, the proximal fixed region 28 may be located laterally outward of the side edge 3c of the absorbent assembly 3. When the proximal fixed region 28 is located to entirely overlap the absorbent assembly 3, the laterally distal edge of the proximal fixed region 28 may be coincident with the side edge 3c of the absorbent assembly 3, or the laterally distal edge of the proximal fixed region 28 may be laterally inward of the side edge 3c of the absorbent assembly 3 by a prescribed distance.

The elasticized region G of the embodiment has the plurality of columns R of bonds substantially equally spaced in the lateral direction Y While not shown in Fig. 2, a large number of columns R of bonds are provided at a substantially regular interval in a region from near one of the pair of distal fixed regions 27 and 27 to near the other. In the region between the pair of proximal fixed regions 28 are there no elastic members 24, or elastic members 24 are disposed there, but the region is de-elasticized by, for example, cutting the elastic members.

The outer cover 2 has a non-bonded region 25 where the outer sheet 22 and the inner sheet 23 are not bonded together between laterally adjacent bonds 26. The non-bonded region 25 is continuous in the longitudinal direction X in the elasticized region G. Having said that, the non-bonded region 25 is allowed to have a minute bond at which the elastic member 24 and the sandwiching sheets 22 and 23 are bonded via an adhesive applied directly to the elastic member 24. The minute bond may be so weak as to be debonded when folds are formed as will be described later. As illustrated in Fig. 3, the elasticized region G of the embodiment is configured such that the outer sheet 22 protrudes away from the wearer's skin between every pair of adjacent columns R of bonds, i.e., in the non-bonded regions 25 on contraction of the unshown elastic members disposed in a stretched state between the outer sheet 22 and the inner sheet 23, whereby a plurality of folds 29 running in the longitudinal direction X are created. The folds 29 provide air passageways to help the humidity inside the diaper 1 to escape thereby to reduce inside stuffiness. The outer cover 2 may be configured such that the inner sheet 23 protrudes toward the wearer's skin in the non-bonded regions 25.

Fig. 4a shows a first embodiment of the waist elasticized region G1 and the lower elasticized region G2 according to the present invention. As shown, a single column R of bonds lining up in the longitudinal direction X is composed of a column R1 in the waist elasticized region G1 and a column R2 in the lower elasticized region G2. The column R extends in the longitudinal direction X through the waist elasticized region G1 (inclusive of the waist edge elasticized region G3) and the lower elasticized region G2.

According to this embodiment, the longitudinal positions of the individual bonds 26a of the waist elasticized region G1 are substantially the same among all the columns R1. The individual elastic members 24 run in the lateral direction Y between longitudinally adjacent bonds 26a in every column R1. Every elastic member 24 is sandwiched in between the outer sheet 22 and the inner sheet 23. The bonds formed in the waist elasticized region G1 will also be called first bonds 26a, and the bonds formed in the waist edge elasticized region G3, which is included in the waist elasticized region G1, will also be called first end bonds a.

Similarly, the longitudinal positions of the individual bonds 26b of the lower elasticized region G2 are substantially the same among all the columns R2. The individual elastic members 24 run in the lateral direction Y between longitudinally adjacent bonds 26b in every column R2. Every elastic member 24 is sandwiched in between the outer sheet 22 and the inner sheet 23. The bonds formed in the lower elasticized region G2 will also be called second bonds 26b.

The outer cover 2 of the diaper 1 has a lower bond strength in the waist elasticized region G1 than in the lower elasticized region G2. That is, the outer cover 2 satisfies inequality (1):

$$B1 < B2 \quad \cdots\cdots\cdots\cdots\cdots\cdots\cdots\cdots\cdots \quad \text{Ineq. (1)}$$

wherein B1 is the bond strength in the waist elasticized region G1; and B2 is the bond strength in the lower elasticized region G2. As used herein, the term "bond strength" refers to the bonding strength between the outer sheet 22 and the inner sheet 23 at the bonds 26.

Accordingly, the outer sheet 22 and the inner sheet 23 are more likely to peel apart from each other at the bonds 26 in the waist elasticized region G1 than in the lower elasticized region G2. In the present embodiment, the first bonds 26a and the second bonds 26b are equal in shape and size in plan view as illustrated in Fig. 4a and therefore have the same area per bond. However, the bond strength per first bond 26a is smaller than that per second bond 26b.

The absorbent member 33 of the diaper 1 increases in weight while worn on absorbing a body fluid, such as urine, discharged from the wearer. The outer cover 2 of the embodiment, which satisfies Ineq. (1) above, is prevented from sagging due to the increased weight of the absorbent member 33 for the following reasons. Since the outer sheet 22 and the inner sheet 23 are less likely to be debonded from each other in the lower elasticized region G2 than in the waist elasticized region G1, the elastic members 24 in the lower elasticized region G2 remain effective in elasticizing the lower elasticized region G2 and capable of stably hold the absorbent member 33 having the increased weight.

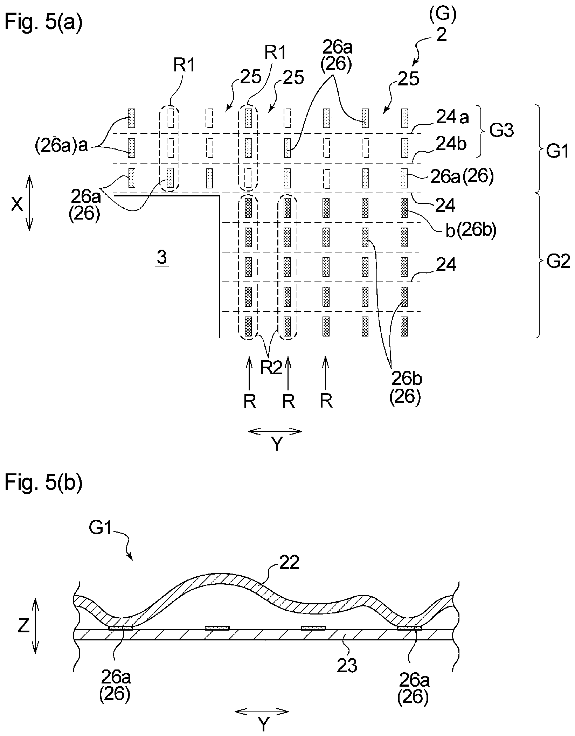

On the other hand, the waist elasticized region G1 is more likely to allow the outer sheet 22 and the inner sheet 23 to be debonded than the lower elasticized region G2 and therefore exhibits excellent conformability to the wearer's waist. These effects will be elaborated by referring to Figs. 4 and 5.

Fig. 4a is an enlarged plan of the elasticized region G, showing the arrangement of bonds before a wearer moves. Fig. 4b is a cross-section of the waist elasticized region G1 before a wearer moves, taken along the lateral direction Y. Fig. 5a is an enlarged plan of the elasticized region G, showing the arrangement of bonds after a wearer moves. Fig. 5b is a cross-section of the waist elasticized region G1 after a wearer moves, taken along the lateral direction Y In Fig. 5a, the bonds at which the outer sheet 22 and the inner sheet 23 are debonded are delineated by a dotted line. According as a wearer walks or changes the position, the outer sheet 22 and the inner sheet 23 are debonded in a part of the first bonds 26a of the waist elasticized region G1. That is, as illustrated in Figs. 5a and 5b, the outer sheet 22 and the inner sheet 23 separate from each other at some first bonds 26a. On debonding the two sheets constituting the outer cover 2 in that way, the conformability of the inner sheet 23 to the wearer's skin improves as compared with before the debonding. As a result, rubbing or chafing of the inner sheet 23 against the skin is reduced. Considering that, in particular, the waist elasticized region G1 is liable to chafe the skin of the wearer's waist with the wearer's movement, the outer cover 2 of the embodiment is very effective in reducing chafing against that part of the wearer's body. The outer cover 2 of the embodiment not only reduces skin chafing during use but also feels soft to provide wearer comfort.

As stated above, the outer cover 2 satisfying Ineq. (1) achieves both prevention of sagging of the diaper 1 after fluid acquisition and conformability to the skin.

The bond strength in the waist elasticized region G1 and the lower elasticized region G2 are measured by the following method. Method for measuring bond strength in waist elasticized region and lower elasticized region:

A specimen is cut out of the elasticized region G of the diaper 1. Specifically, a side portion E of the outer cover 2 is cut in the longitudinal direction along the crests of laterally adjacent two folds 29 to make a specimen having a column R of bonds between the crests. The resulting specimen contains the waist elasticized region G1 and the lower elasticized region G2. Similarly, the middle portion F of the outer cover 2 is cut in the longitudinal direction along the crests of laterally adjacent two folds 29 to make a specimen having a column R1 of bonds between the crests of the folds. The resulting specimen contains only the waist elasticized region G1. The outer sheet and the inner sheet at the proximal end (closer to the crotch section C) of each specimen are clamped in jaws of a tensile tester Autograph AGS50A (from Shimadzu Corp.) and pulled at 180° at a rate of 300 mm/min. The peel strengths of the first bonds 26a or the second bonds 26b of the specimen are measured until the two sheets are completely separated apart. The measurements of five first bonds 26a are averaged to give the bond strength of the waist elasticized region G1. The measurements of five second bonds 26b are averaged to give the bond strength of the lower elasticized region G2.

When the waist elasticized region G1 has first bonds 26a having different bond strengths, the peel strength of the first end bonds a in the waist edge elasticized region G3 is taken as the peel strength of the waist elasticized region G1. The first end bonds a are those located between the first elastic member 24a and the second elastic member 24b.

When the lower elasticized region G2 has second bonds 26b having different bond strengths, the peel strength of the second bonds 26b closest to the waist opening 5 is taken as the bond strength of the lower elasticized region G2. The second bonds 26b closest to the waist opening 5 are those located between the elastic member closest to the waist opening 5 and the second closest one. The second bond 26b closest to the waist opening 5 will also be called a second end bond b.

When the number of the first end bonds a or the second end bond b is smaller than five, an average peel strength of the largest possible number of the bonds a or b is obtained.

In order to ensure the above effects, the bond strength ratio of the waist elasticized region G1 to the lower elasticized region G2 is preferably 0.10 or higher, more preferably 0.20 or higher, preferably 0.95 or lower, more preferably 0.90 or lower, and preferably 0.10 to 0.95, more preferably 0.20 to 0.90. With a view to further improving the conformability to the skin in the waist elasticized region G1, the bond strength of the waist elasticized region G1 is preferably 0.01 N or greater, more preferably 0.02 N or greater, preferably 0.36 N or smaller, more preferably 0.30 N or smaller, and preferably 0.01 to 0.36 N, more preferably 0.02 to 0.30 N. With a view to further improving the capability of holding the absorbent member 33 after fluid absorption, the bond strength of the lower elasticized region G2 is preferably 0.05 N or greater, more preferably 0.075 N or greater, preferably 0.6 N or smaller, more preferably 0.5 N or smaller, and preferably 0.05 to 0.6 N, more preferably 0.075 to 0.5 N.

With the view to further improving the conformability of the waist elasticized region G1 to the skin, the middle portion F of the waist elasticized region G1 preferably has a lower bond strength than the side portions E of the region G1. Such a configuration facilitates debonding between the outer sheet 22 and the inner sheet 23 of the waist elasticized region G1 in the middle portion F caused by the increased weight of the absorbent member 33 after fluid absorption. To ensure this effect, the bond strength of the waist elasticized region G1 in the middle portion F is preferably 10% or more, more preferably 20% or more, preferably 95% or less, more preferably 90% or less, and preferably 10% to 95%, more preferably 20% to 90%, of that in the side portions E. For the same purpose, the bond strength of the waist elasticized region G1 in the middle portion F is preferably 0.01 N or greater, more preferably 0.02 N or greater, preferably 0.36 N or smaller, more preferably 0.30 N or smaller, and preferably 0.01 to 0.36 N, more preferably 0.02 to 0.30 N. The bond strength of the waist elasticized region G1 in the side portions E is preferably 0.01 N or greater, more preferably 0.02 N or greater, preferably 0.36 N or smaller, more preferably 0.30 N or smaller, and preferably 0.01 to 0.36 N, more preferably 0.02 to 0.30 N. It is preferred that the bond strength of the middle portion F be lower than that of the side portions E within the above ranges.

The bond strength of the waist elasticized region G1 in the middle portion F is the average peel strength at the first bonds 26a in the middle portion F. The bond strength of the waist elasticized region G1 in the side portions E is the average peel strength at the first bonds 26a in the side portions E. These average peel strengths are obtained by the same method as "method for measuring bond strength in waist elasticized region and lower elasticized region" described above.

In the present embodiment, the bonds 26 in the elasticized region G are discrete in the longitudinal direction. Specifically, every bond 26 is formed between longitudinally adjacent elastic members 24 with no overlaps with any elastic member 24. Such an arrangement of bonds 26 facilitates separation between the outer sheet 22 and the inner sheet 23, thereby to further improve the conformability to the skin.

In the embodiment, the absorbent member 33 has overlap regions 38 and 38 that overlap the lower elasticized regions G2 and G2 in the longitudinal direction X in each of the front section A and the rear section B and a non-overlap region 39 in the crotch section C that does not overlap the lower elasticized regions G2 and G2 in the longitudinal direction.

With a view to further improving the capability of holding the absorbent member 33 after fluid absorption, it is preferred that the overlap regions 38 and 38 have a larger basis weight of an absorbent material than the non-overlap region 39. When the absorbent member is so designed, the peel strength at the second bonds 26b of the overlap regions 38 further increases. The absorbent material is exemplified by pulp fiber and an absorbent polymer. To ensure the above effect, the basis weight of the absorbent material in the overlap regions 38 and 38 is preferably 1.05 times or more, more preferably 1.10 times or more, preferably 1.7 times or less, more preferably 1.5 times or less, and preferably 1.05 to 1.7 times, more preferably 1.10 to 1.5 times, that in the non-overlap region 39.

With a view to concentrating a body fluid such as urine in the overlap regions 38 and 38 thereby to further improve the capability of holding the absorbent member 33 after fluid acquisition, the absorbent member 33 preferably has a longitudinally extending fiber-free region 37 in the non-overlap region 39. The absorbent member 33 used in the embodiment has a single fiber-free region 37 in the lateral middle of the non-overlap region 39. Instead of the single fiber-free region 37, the absorbent member 33 may have a plurality of fiber-free regions spaced in the lateral direction Y As used herein, the term "fiber-free region" means a hole or slit going through the thickness of the absorbent core (hereinafter described) of the absorbent member 33.

In order to further improve the capability of holding the absorbent member 33 after fluid acquisition and the conformability of the waist elasticized region G1 to the skin, the individual elastic members 24 in the waist elasticized region G1 preferably have a smaller extensional stress than those in the lower elasticized region G2. By this design, the lower elasticized region G2 keeps holding the absorbent member 33 more stably by the extensional stress of the elastic members 24 disposed therein even after the absorbent member 33 increases in weight.

The extensional stress can be determined as follows.

Method for measuring extensional stress:

The diaper is cut to remove the portion blow the lower end of the side seams 4. The portion corresponding to the front or rear section A or B is cut along the boundary between the waist elasticized region G1 and the lower elasticized region G2 parallel to the edge 51 of the waist opening 5 to make a specimen having the waist elasticized region G1 and a specimen having the lower elasticized region G2. Each specimen is set in a tensile tester (e.g., RTA-100 from Orientec Co., Ltd.) at an initial jaw separation of 30 mm with its lengthwise direction coincident with the tensile direction. The specimen is 100% stretched (extended) at a rate of 300 mm/min and then allowed to contract at the same rate until the tensile stress becomes zero. The tensile stress varying with the extension and contraction is recorded to prepare a curve of tensile strength vs. stretched length. The above expression "100% stretched" means a state of the specimen stretched until the folds 29 disappear. The tensile stress at the 100% stretch is defined to be a 100% stretch stress. A tensile stress corresponding to 70% of the 100% stretch stress during the contraction is obtained from the curve, which is defined to be an extensional stress.

With the view to further improving the capability of holding the absorbent member 33 after fluid absorption and the conformability of the waist elasticized region G1 to the skin, the extensional stress of the elastic members 24 in the waist elasticized region G1 is preferably 10% or more, more preferably 20% or more, preferably 95% or less, more preferably 90% or less, and preferably 10% to 95%, more preferably 20% to 90%, of that in the lower elasticized region G2. With the same view, the extensional stress of the elastic members 24 in each of the waist elasticized region G1 and the lower elasticized region G2 is preferably as follows. The extensional stress of the elastic members 24 in the waist elasticized region G1 is preferably 0.05 N/cm or higher, more preferably 0.10 N/cm or higher, preferably 0.90 N/cm or lower, more preferably 0.70 N/cm or lower, and preferably 0.05 to 0.90 N/cm, more preferably 0.10 to 0.70 N/cm. The extensional stress of the elastic members 24 in the lower elasticized region G2 is preferably 0.25 N/cm or higher, more preferably 0.5 N/cm or higher, preferably 2.5 N/cm or lower, more preferably 2.0 N/cm or lower, and preferably 0.25 to 2.5 N/cm, more preferably 0.5 to 2.0 N/cm, provided that it is higher than that in the waist elasticized region G1.

In order for the waist elasticized region G1 and the lower elasticized region G2 to have different extensional stresses for their elastic members 24 as discussed above, the elastic members 24 of the waist elasticized region G1 preferably have a smaller fineness than those of the lower elasticized region G2. Specifically, the fineness of the elastic members 24 of the waist elasticized region G1 is preferably 10% or more, more preferably 20% or more, preferably 90% or less, more preferably 80% or less, and preferably 10% to 90%, more preferably 20% to 80%, of that of the lower elasticized region G2. More specifically, the fineness of the elastic members 24 of the waist elasticized region G1 is preferably 100 dtex or larger, more preferably 120 dtex or larger, preferably 700 dtex or smaller, more preferably 650 dtex or smaller, and preferably 100 to 700 dtex, more preferably 120 to 650 dtex. The fineness of the elastic members 24 of the lower elasticized region G2 is preferably 300 dtex or larger, more preferably 350 dtex or larger, preferably 1000 dtex or smaller, more preferably 900 dtex or smaller, and preferably 300 to 1000 dtex, more preferably 350 to 900 dtex, provided that it is larger than that of the waist elasticized region G1.

In order to apply a uniform extensional stress of the elastic members 24 to the waist elasticized region G1 and the lower elasticized region G2, the longitudinal interval P10 (Fig. 4a) of the elastic members in the waist elasticized region and the longitudinal interval P11 (Fig. 4a) of the elastic members in the lower elasticized region are preferably equal. Such a configuration prevents the outer cover 2 from loosening and further improves the conformability to the skin of the waist elasticized region G1. With the view of preventing the outer cover 2 from loosening more effectively and facilitating formation of the folds 29, the longitudinal intervals P10 and P11 of the elastic members 24 in the waist elasticized region G1 and the lower elasticized region G2, respectively, are preferably 3 mm or more, more preferably 4 mm or more, preferably 12 mm or less, more preferably 10 mm or less, and preferably 3 to 12 mm, more preferably 4 to 10 mm.

In order for the waist elasticized region G1 to have a larger extensional stress to further improve the conformability to the skin, the longitudinal distance between the elastic members 24 in the waist elasticized region G1 is preferably longer than that in the lower elasticized region G2. Specifically, the longitudinal interval P10 (Fig. 4a) of the elastic members 24 in the waist elasticized region G1 is preferably 105% or more, more preferably 110% or more, preferably 800% or less, more preferably 600% or less, and preferably 105% to 800%, more preferably 110% to 600%, of the interval P11 (Fig. 4a) of the elastic members 24 in the lower elasticized region G2. The longitudinal interval P10

(Fig. 4a) of the elastic members 24 in the waist elasticized region G1 is preferably 4 mm or more, more preferably 5 mm or more, preferably 15 mm or less, more preferably 12 mm or less, and preferably 4 to 15 mm, more preferably 5 to 12 mm. The longitudinal interval P11 (Fig. 4a) of the elastic members 24 in the lower elasticized region G2 is preferably 3 mm or more, more preferably 4 mm or more, preferably 12 mm or less, more preferably 10 mm or less, and preferably 3 to 12 mm, more preferably 4 to 10 mm, provided that it is smaller than the interval P10 of the elastic members 24 of the waist elasticized region G1.

Since the first bonds 26a and the second bonds 26b in the embodiment are the same in area per bond but different in bond strength per bond, the waist elasticized region G1 and the lower elasticized region G2 may be designed to be different in bond strength. When, for example, the bonds 26 are formed by fusion bonding, it is preferred for forming bonds having different bond strengths that the bonds 26, i.e., fusion bonds 26 of the waist elasticized region G1 have a smaller cross-sectional fusion area than those of the lower elasticized region G2.

In the individual bonds 26 formed by fusion, the resin constituting fibers of either one of or both the outer sheet 22 and the inner sheet 23 are in a molten and solidified state. Every bond 26 has a higher density than other regions of the outer and inner sheets, i.e., regions other than the bonds. Such bonds 26 are preferably thermal fusion bonds formed by integrally heating the outer sheet 22 and the inner sheet 23 under pressure, in which the two sheets are bonded together as a result of melting and subsequent solidification of the resin constituting the fibers making up either one or both of the two sheets. It is desirable that both the outer sheet 22 and the inner sheet 23 be melted and solidified at the individual bonds 26. Such bonds 26 may be formed by, for example, thermal embossing or ultrasonic embossing.

The cross-sectional fusion area of the individual bonds 26 can be measured as follows.

Method for measuring cross-sectional fusion area of bond:

A specimen having a single first bond 26a or second bond 26b, the bond strength of which is to be measured, is cut out by cutting the waist elasticized region G1 or the lower elasticized region G2, respectively, of the outer cover in the longitudinal direction X and the lateral direction Y The longitudinally cut cross-section of the specimen is observed under a scanning electron microscope (SEM) (e.g., JSM-IT100 from JEOLLtd.) at a magnification of 100 to 200 times. The bond in the field of view is observed as a cohesive film-like portion with no fibrous form remaining. The portion other than the bond is observed as retaining a fibrous form. The cohesive portion in the SEM image is outlined, and the area of the outlined portion is measured. The measurement is taken for randomly chosen three bonds in each of the waist elasticized region G1 and the lower elasticized region G2. The average of the three measurements is taken as the longitudinal cross-sectional fusion area. A lateral cross-sectional fusion area is obtained in the same manner. The sum of the longitudinal cross-sectional fusion area and the lateral cross-sectional fusion area is defined to be the cross-sectional fusion area of the bond.

In order to make a difference in bond strength between the waist elasticized region G1 and the lower elasticized region G2 more certainly, it is preferred that the cross-sectional fusion area of the bonds in the waist elasticized region G1 be 5% or more, more preferably 10% or more, preferably 95% or less, more preferably 90% or less, and preferably 5% to 95%, more preferably 10% to 90%, of that in the lower elasticized region G2.

To facilitate formation of the bonds with different bond strengths, the heating (fusion) temperature for the formation of the first bonds 26a and that for the formation of the second bonds 26b are preferably different. It is more preferred that the heating temperature for the formation of the first bonds 26a be lower than that for the formation of the second bonds 26b. When the bonds 26a and 26b are formed by embossing, for example, the heating temperature for the formation of the first bonds 26a is preferably lower than that for the formation of the second bonds 26b by 3° to 5°C.

To facilitate formation of the bonds by fusion, the outer sheet 22 and the inner sheet 23 preferably contain sheath/core conjugate fibers composed of a sheath component and a core component which are preferably different from each other. The sheath/core conjugate fibers are preferably thermally bondable fibers, specifically fibers composed of a plurality of thermoplastic polymer materials. Examples of the thermoplastic polymer materials include polyolefins, such as polyethylene and polypropylene, polyesters, such as polyethylene terephthalate, and polyamides. The sheath/core conjugate fibers are exemplified by those having a polyethylene terephthalate or polypropylene core and a polyethylene sheath. For easy bond formation, the sheath component preferably has a lower melting point than the core component.

With the view of forming the bonds having different bond strengths, when, for example, the bonds 26 are formed by hot-melt adhesive bonding, the depth of penetration of the hot-melt adhesive at the bonds 26 is preferably smaller in the waist elasticized region G1 than in the lower elasticized region G2.

The depth of penetration of hot-melt adhesive at the bonds 26 can be determined by the method below.

Method for measuring penetration depth of hot-melt adhesive at bond:

Specimens showing a cross-sectional cut surface are made in the same manner as in the "method for measuring cross-sectional fusion area of bond" described above. The cross-sectional cut surface of the specimen is photographed using a microscope VHX1000 from Keyence at a magnification of 200 times to obtain an image of the field of view. The portion of the image where the hot-melt adhesive has penetrated, which is observed as a transparent shiny portion, is outlined, and the area of the outlined portion is measured. The measurement is taken at five end bonds a, and the average of the measured areas is defined to be the penetration depth of hot-melt adhesive at bonds a of the waist elasticized region G1. The same measurements are taken for five second bonds 26b, and the average of the measured areas is defined to be the penetration depth of hot-melt adhesive at bonds of the lower elasticized region G2.

In order to make a difference in bond strength between the waist elasticized region G1 and the lower elasticized region G2 more certainly, it is preferred that the penetration depth of hot-melt adhesive at bonds a in the waist elasticized region G1 be 5% or more, more preferably 10% or more, preferably 95% or less, more preferably 90% or less, and preferably 5% to 95%, more preferably 10% to 90%, of that at bonds 26b in the lower elasticized region G2.

To facilitate formation of the bonds with different bond strengths, the temperature of applying a hot-melt adhesive for the formation of the first bonds 26a and that for the formation of the second bonds 26b are preferably different. It is more preferred that the temperature of adhesive application for the formation of the first bonds 26a be lower than that for the formation of the second bonds 26b. Specifically, the temperature of adhesive application for the formation of the first bonds 26a is preferably lower than that for the formation of the second bonds 26b by 3° or more, more preferably by 5°C or more.

The hot-melt adhesive may be applied by a known method, such as coater method, gravure method, flexo method, or bead method. Bead method is preferred for prevention of pulp fiber flying and penetration depth control.

Examples of useful adhesives include styrene- (e.g., SIS, SBS, or SEBS) based or polyolefin-based hot melt adhesives. A pressure-sensitive SIS-based hot-melt adhesive is particularly preferred with a view to preventing elastic members from being drawn and preventing strike-through of the adhesive.

Other embodiments of the elasticized region G according to the present invention will then be described. The description of the other embodiments will generally be confined to the differences from the first one illustrated in Figs. 1 through 5. It should be understood that identical or corresponding elements throughout the drawings are given identical reference numerals and will not be elaborated upon. Unless the context specifically states otherwise, the description of the first embodiment applies equally to the other embodiments.

The aforementioned relationship represented by Ineq. (1) is established by designing the bond strength per bond in the waist elasticized region G1 to be lower than that in the lower elasticized region G2 as discussed above. Otherwise, Ineq. (1) is also established by designing the bond area per unit area of the waist elasticized region G1 to be smaller than that of the lower elasticized region G2 as will be elaborated below.

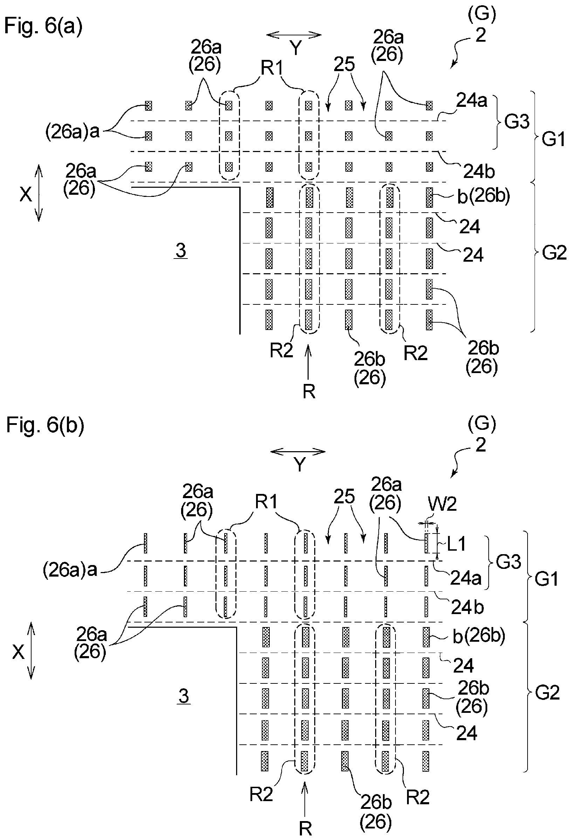

Figs. 6a and 6b show a second and a third embodiment, respectively, in which the waist elasticized region G1 has a smaller bond area per unit area thereof than the lower elasticized region G2.

In the second embodiment illustrated in Fig. 6a, the individual bonds 26 in the waist elasticized region G1 are shorter in the longitudinal direction X than those in the lower elasticized region G2. As illustrated, the first bonds 26a have the same lateral dimension (or width) as the second bonds 26b but a smaller longitudinal dimension (or length) than the second bonds 26b. Accordingly, the area of the individual first bonds 26a is smaller than that of the individual second bonds 26b.

In the third embodiment illustrated in Fig. 6b, the individual bonds 26 in the waist elasticized region G1 have a smaller width than those in the lower elasticized region G2. In this embodiment, the first bonds 26a have the same longitudinal dimension (or length) as the second bonds 26b but a smaller width than the second bonds 26b. Accordingly, the area of the individual first bonds 26a is smaller than that of the individual second bonds 26b.

When the waist elasticized region G1 has a smaller bond area per unit area than the lower elasticized region G2 as in the second and third embodiments, the bond area per unit area of the waist elasticized region G1 is preferably 5% or more, more preferably 10% or more, preferably 95% or less, more preferably 90% or less, and preferably 5% to 95%, more preferably 10% to 90%, of that of the lower elasticized region G2. The bond area per unit area of the waist elasticized region G1 or the lower elasticized region G2 is the ratio of the total area of the first bonds 26a or the second bonds 26b, respectively, to the area of the respective specimen prepared in the same manner as in the "method for measuring bond strength in waist elasticized region and lower elasticized region" described above.

In the first to third embodiments the first bonds 26a and the second bonds 26b are arranged regularly at a prescribed interval in both the longitudinal direction X and the lateral direction Y as illustrated in Figs. 4, 6a, and 6b. When the bonds are arranged regularly and discretely, preferred repeat distances in the longitudinal direction X and the lateral direction Y are as follows. The repeat distance of the first bonds 26a and that of the second bonds 26b may be the same or different in the longitudinal direction X or the lateral direction Y. The repeat distances of bonds in the present description are measured with the elasticized region G being in the laterally fully stretched state, i.e., in its uncontracted condition.

With the view to further improving the conformability to the skin, the repeat distances P1 and P3 (see Fig. 4a) of the bonds 26 in the lateral direction Y are each preferably 2 mm or longer, more preferably 4 mm or longer, preferably 12 mm or shorter, more preferably 10 mm or shorter, and preferably 2 to 12 mm, more preferably 4 to 10 mm. The term "repeat distance in the lateral direction Y" as used for the bonds 26 denotes a distance as measured between the lateral center of a first bond 26a and the lateral center of a laterally adjacent first bond 26a or between the lateral center of a second bond 26b and the lateral center of a laterally adjacent second bond 26b.

Wit the same view, the repeat distances P2 and P4 (see Fig. 4a) of the bonds 26 in the longitudinal direction X are each preferably 1 mm or longer, more preferably 2 mm or longer, preferably 12 mm or shorter, more preferably 10 mm or shorter, and preferably 1 to 12 mm, more preferably 2 to 10 mm. The term "repeat distance in the longitudinal direction X" as used for the bonds 26 denotes a distance as measured between the longitudinal center of a first bond 26a and the longitudinal center of a longitudinally adjacent first bond 26a or between the longitudinal center of a second bond 26a and the longitudinal center of a longitudinally adjacent second bond 26a.

In the first to third embodiments, the bonds 26 are discretely formed along the longitudinal and lateral directions. In such arrangements of bonds, the individual bonds 26, inclusive of bonds 26a and 26b, preferably have the dimensions described below with the view of further improving conformability of the waist elasticized region G1 to the skin and facilitating debonding between the outer sheet 22 and the inner sheet 23.

The longitudinal dimension of the individual first bonds 26a, i.e., length L1 (Fig. 4a) is preferably 5% or more, more preferably 10% or more, preferably 100% or less, more preferably 95% or less, and preferably 5% to 100%, more preferably 10% to 95%, of that of the individual second bonds 26b, i.e., length L3 (Fig. 4a).

The lateral dimension of the individual first bonds 26a, i.e., width W1 (Fig. 4a) is preferably 5% or more, more preferably 10% or more, preferably 100% or less, more preferably 95% or less, and preferably 5% to 100%, more preferably 10% to 95%, of that of the individual second bonds 26b, i.e., width W3 (Fig. 4a).

The widths W1 and W3 (Fig. 4a) of the bonds 26 are each preferably 0.1 mm or greater, more preferably 0.5 mm or greater, preferably 4 mm or smaller, more preferably 3 mm or smaller, and preferably 0.1 to 4 mm, more preferably 0.5 to 3 mm. The lengths L1 and L3 (Fig. 4a) of the bonds 26 are each preferably 0.5 mm or greater, more preferably 1 mm or greater, preferably 10 mm or smaller, more preferably 8 mm or smaller, and preferably 0.5 to 10 mm, more preferably 1 to 8 mm.

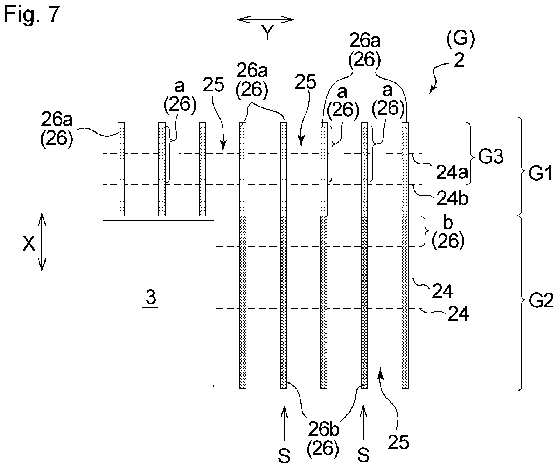

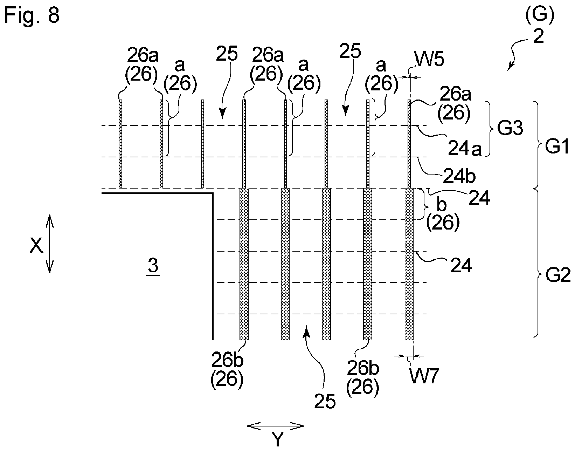

The bonds 26 of the outer cover 2 may be continuous in the longitudinal direction as illustrated in Figs. 7 and 8. The embodiments shown in Figs. 7 and 8 are the same as the foregoing embodiments except for the following differences, so that the description about the foregoing embodiments equally applies to the embodiments of Figs. 7 and 8 unless inconsistent with the context.

The elasticized region G of a fourth embodiment has first bonds 26a and second bonds 26b of the same width. As illustrated in Fig. 7, the individual bonds 26a continue to the individual bonds 26b to form a plurality of longitudinally continuous and laterally spaced columns S of bonds. In the fourth embodiment, the first bonds 26a have a smaller bond strength per unit area than the second bonds 26b, whereby the waist elasticized region G1 has a lower bond strength than the lower elasticized region G2. The bond strength as referred to here can be measured in the same manner as in the "method for measuring bond strength in waist elasticized region and lower elasticized region", except that the specimen has a longitudinal dimension, i.e., a length of 10 mm.

In the elasticized region G of a fifth embodiment, which is illustrated in Fig. 8, the first bonds 26a has a width W5 that is smaller than a width W7 of the second bonds 26b, whereby the waist elasticized region G1 has a smaller bond area per unit area than the lower elasticized region G2.

To facilitate debonding between the outer sheet 22 and the inner sheet 23 at the first bonds 26a of the waist elasticized region G1 in the first to fifth embodiments, it is preferred for the sheets 22 and 23 composing the outer sheet 2 to have any one or more of the following structures.

Structure (1): One or both of the inner sheet 23 and, if any, a sheet disposed on the skin facing side of the inner sheet 23 to make up the outer cover 2 have higher hydrophilicity than the outer sheet 22.
Structure (2): The outer sheet 22 and the inner sheet 23 have different contents of a softening agent.
Structure (3): The inner sheet 23 has a higher coefficient of friction than the outer sheet 22.
Structure (4): The inner sheet 23 has a higher fiber density than the outer sheet 22.

In structure (1), a sheet composing the outer cover 2 which is disposed closer to the skin than the elastic members 24 is more hydrophilic than the outer sheet 22. Any sheet composing the outer cover 2 which is disposed closer to the skin than the elastic members 24, such as the inner sheet 23, will hereinafter be called an inward sheet. Not only the inner sheet 23 but any sheet disposed closer to the skin than the inner sheet 23 is an inward sheet. An inward sheet other than the inner sheet 23 is exemplified by a folded-over portion of the outer sheet 22 or the inner sheet 23 that is folded around the edge 51 of the waist opening 5 toward the skin facing side of the inner sheet 23. The folded-over portion is usually bonded to the skin facing side of one or both of the inner sheet 23 and the absorbent assembly 3 with adhesive applied to the entire area thereof or in parts.

The hydrophilicity of a sheet can be represented in terms of contact angle with water. A contact angle with water may be measured by, for example, the method described in JP 2015-142721A. With the view of further facilitating debonding between the outer sheet 22 and the inner sheet 23 at the bonds 26, the inward sheet preferably has a water contact angle of 50° or more, more preferably 70° or more, preferably 130° or less, more preferably 120° or less, and preferably 50° to 130°, more preferably 70° to 120°. With the same view, the outer sheet 22 preferably has a water contact angle of 90° or more, more preferably 110° or more, preferably 180° or less, more preferably 179° or less, and preferably 90° to 180°, more preferably 110° to 179°. The larger the water contact angle of the outer sheet, the more effective in preventing moisture condensation. Specifically, the water contact angle of the outer sheet is preferably 180° or smaller.

The hydrophilicity of a sheet is adjustable by the content of hydrophilic fibers, such as cellulosic fibers. Examples of hydrophilic fibers include cellulosic fibers, such as pulp, cotton, rayon, Lyocell, and Tencel, and hydrophilized synthetic fibers made of thermally fusible resins. In order to achieve structure (1), it is preferred for at least one of the outer sheet 22 and the inner sheet 23 to contain cellulosic fibers. It is more preferred that the inward sheet contains cellulosic fibers. It is also more preferred that the outer sheet 22 having the above described folded-over portion contains cellulosic fibers.

In order for the inward sheet to have higher hydrophilicity than the outer sheet 22, it is preferred for the inward sheet to have a higher cellulosic fiber content than the outer sheet 22. For instance, the cellulosic fiber content of the inner sheet 23 is preferably 5 mass% or more, more preferably 10 mass% or more, preferably 100 mass% or less, more preferably 80 mass% or less, and preferably 5 to 100 mass%, more preferably 10 to 80 mass%, provided that it is higher than that of the outer sheet 22. The cellulosic fiber content of the outer sheet 22 is preferably 0 mass% or more, more preferably 5 mass% or more, preferably 50 mass% or less, more preferably 40 mass% or less, and preferably 0 to 50 mass%, more preferably 5 to 40 mass%.

In structure (2), the outer sheet 22 and the inner sheet 23 have different contents of a softening agent. With the view of improving conformability to the skin, the softening agent content of the inner sheet 23 is preferably higher than that of the outer sheet 22. The softening agent may be externally applied by spraying or otherwise or incorporated into constituent fibers in the production of the sheet.

The softening agent content of the inner sheet 23 is preferably 0.01 mas% or more, more preferably 0.05 mass% or more, preferably 7 mass% or less, more preferably 5 mass% or less, and preferably 0.01 to 7 mass%, more preferably 0.05 to 5 mass%, provided that it is higher than that of the outer sheet 22. The softening agent content of the outer sheet 22 is preferably 0.005 mass% or more, more preferably 0.01 mass% or more, preferably 6 mass% or less, more preferably 4 mass% or less, and preferably 0.005 to 6 mass%, more preferably 0.01 to 4 mass%. The softening agent content of each sheet is determined by the method below. A specimen is cut out of a non-bonded region of the sheet being analyzed where the sheet is not bonded to itself or another sheet by, for example, hot-melt adhesive bonding or fusion bonding. After being dried in an electric dryer at 60°C for 1 hour, the specimen is weighed to give an initial mass. The specimen is immersed in acetone for 2 hours, dried spontaneously for one week, dried in an electric dryer at 60°C for 1 hour, and weighted again to give a softening agent-free mass. The softening agent-free mass is subtracted from the initial mass, the difference is divided by the softening agent-free mass, and the quotient is multiplied by 100 to give the softening agent content (%).

Examples of useful softening agents include wax emulsions, reactive softening agents, silicone compounds, fatty acid amide compounds, such as diamide derivatives, and surfactants.

In structure (3), the inner sheet 23 has a higher friction coefficient than the outer sheet 22. This helps the inner sheet 23 to keep in contact with the skin so that the outer sheet 22 and the inner sheet 23 may be separated more easily at the first bonds 26a of the waist elasticized region G1. The friction efficient of a sheet is represented in terms of mean deviation of friction coefficient (MMD) measured using KESFB4-AUTO-A from Kato Tech Co., Ltd. in accordance with the method described in Kawabata, Hideo, Fuai Hyouka no Hyoujunka to Kaiseki (Standardization and Analysis of Hand Evaluation), Japanese Hand Evaluation and Standardization Committee (HESC), The Textile Machinery Soc. of Japan, 2nd Ed. (Jul. 10, 1980).

Method for determining mean deviation of friction coefficient (MMD):

A sample measuring 20 mm by 20 mm is cut out of each sheet composing the outer cover 2. When a sheet being tested is short of that size, the size of the sample may be altered appropriately. The sample is clamped on a flat metal plate surface. A contact sensor is pressed onto the sample at a contact force of 49 cN, and the sample is moved horizontally at a constant speed of 0.1 cm/sec by 2 cm to be given a uniaxial tension of 7.3 cN/cm. The contact sensor is composed of a set of 20 piano wires of 0.5 mm in diameter arranged in parallel and U-shaped with a bottom width of 10 mm. The contact sensor is pressed onto the sample at a contact force of 49 cN. The mean deviation of friction coefficient is represented in terms of MMD value. The measurement is taken in both the MD and CD to obtain $MMD_{MD}$ and $MMD_{CD}$, an average of which obtained by the following expression is taken as a mean deviation of friction coefficient MMD. In the case where the sheet is too small to make specimens for MD and CD measurements, either $MMD_{MD}$ or $MMD_{CD}$ is used.

$$\text{Mean deviation of friction coefficient MMD} = \{(MMD_{MD}^2 + MMD_{CD}^2)/2\}^{1/2}$$

The mean deviation of friction coefficient MMD will hereinafter be simply referred to as MMD.

With the view of further facilitating separation of the sheets, the MMD of the outer sheet 22 is preferably 10% or more, more preferably 20% or more, preferably 95% or less, more preferably 90% or less, and preferably 10% to 95%, more preferably 20% to 90%, of the MMD of the inner sheet 23. With the same view, the MMD of the outer sheet 22 is preferably 0.001 or greater, more preferably 0.002 or greater, preferably 0.012 or smaller, more preferably 0.010 or smaller, and preferably 0.001 to 0.012, more preferably 0.002 to 0.010.

With the same view, the MMD of the inner sheet 23 is preferably 0.002 or greater, more preferably 0.003 or greater, preferably 0.015 or smaller, more preferably 0.012 or smaller, and preferably 0.002 to 0.015, more preferably 0.003 to 0.012.

In structure (4), the inner sheet 23 has a higher fiber density than the outer sheet 22. Specifically, the fiber density of the outer sheet 22 is preferably 10% or higher, more preferably 20% or higher, preferably 90% or lower, more preferably 80% or lower, and preferably 10% to 90%, more preferably 20% to 80%, of that of the inner sheet 23. Specifically, the fiber density of the outer sheet 22 is preferably $0.01\ g/cm^3$ or higher, more preferably $0.02\ g/cm^3$ or higher, preferably $0.15\ g/cm^3$ or lower, more preferably $0.12\ g/cm^3$ or lower, and preferably 0.01 to $0.15\ g/cm^3$, more preferably 0.02 to $0.12\ g/cm^3$. The fiber density of the inner sheet 23 is preferably $0.02\ g/cm^3$ or higher, more preferably $0.03\ g/cm^3$ or higher, preferably $0.17\ g/cm^3$ or lower, more preferably $0.15\ g/cm^3$ or lower, and preferably 0.02 to $0.17\ g/cm^3$, more preferably 0.03 to $0.15\ g/cm^3$.

The diaper 1 will further be elaborated. The side seams 4 are formed by, after the formation of the bonds 26, superposing the distal fixed regions 27 of the front section A and those of the rear section B on each other and bonding the superposed distal fixed regions 27 by thermal embossing. Formation of the bonds 26 may be achieved by ultrasonic sealing or laser bonding as well as thermal embossing. Formation of the side seams 4 may be carried out not only by thermal embossing but by ultrasonic sealing, laser bonding, or adhesive bonding.

The absorbent assembly 3 includes a liquid permeable topsheet 31, a hardly liquid-impermeable backsheet 3, and an absorbent member 33 interposed between these sheets. As used herein, the term "hardly liquid-permeable" is intended to include "liquid impermeable". The absorbent assembly 3 is provided with a pair of standing cuff-forming sheets 34 and 34 along the opposed longitudinal sides thereof. Each standing cuff-forming sheet 34 is provided with an elastic member 35 along near the laterally proximal edge thereof. While the diaper 1 is worn, the elastic member 35 contracts to raise a prescribed width of the cuff-forming sheet from the proximal edge away from the topsheet 31 thereby to form a standing cuff.

Materials making each constituent member of the diaper 1 will then be described. The topsheet 31, backsheet 32, absorbent member 33, and standing cuff-forming sheet 34 may be of any materials conventionally used in this type of absorbent articles. For example, the topsheet 31 may be formed of liquid permeable nonwoven fabric, perforated film, or a laminate thereof. The backsheet 32 may be formed of resin film or a resin film/nonwoven fabric laminate. The absorbent member 33 may be an absorbent core formed of an aggregate of fibrous materials, such as pulp, or an absorbent core having an absorbent polymer dispersed therein and wrapped in a core wrap sheet, such as tissue or water-permeable nonwoven fabric. The standing cuff-forming sheet 34 may be formed of extensible and contractible film, water-repellant nonwoven or woven fabric, or a laminate thereof.

The outer sheet 22 and the inner sheet 23 may be formed of any sheeting materials conventionally used in this type of articles. Nonwoven fabrics are preferably used. Examples of useful nonwoven fabrics include air-through bonded nonwovens, heat-rolled nonwovens, spun-laced nonwovens, spun-bonded nonwovens, melt-blown nonwovens, and complex nonwoven fabrics composed of two or more of these nonwovens. Composite nonwoven fabrics composed of the nonwoven fabric and a resin film are also useful. A nonwoven fabric sheet or a resin film may be disposed on the non-skin facing side of the backsheet in the crotch section C. With the view of further facilitating debonding between the outer sheet 22 and the inner sheet 23 at the first bonds 26a in the waist elasticized region G1, the outer cover 2 is preferably formed of a combination of air-through bonded nonwoven fabric and spun-bonded nonwoven fabric. It is more preferred that the inner sheet 23 be formed of spun-bonded nonwoven fabric and that the outer sheet 22 be formed of air-through bonded nonwoven fabric.

The elastic members 24 and 35 may be made of any known elastic materials of various kinds commonly used in absorbent articles, such as disposable diapers and sanitary napkins, including synthetic rubbers, such as styrene-butadiene, butadiene, isoprene, and neoprene rubbers, natural rubber, EVA, stretch polyolefins, and polyurethanes.

Exemplary forms of the elastic members include a thread with a rectangular, square, circular or polygonal section (e.g., an elastic thread), a string (e.g., elastic tape), or multifilamentous yarn.

While the present invention has been described on the basis of its preferred embodiments, it should be understood that the present invention is not limited thereto, and various changes and modifications can be made as appropriate. The foregoing embodiments may be practiced in appropriate combinations. For instance, the first and second embodiments may be combined such that the first bonds 26a and the second bonds 26b differ in not only bond strength per unit area but bond area per unit area.

The outer cover does not need to be divided into the front panel 2A and the rear panel 2B. For instance, the outer cover may be formed of a single sheet assembly composed of an outer sheet 22 and an inner sheet 23 both continuous from the front section A to the rear section B through the crotch section C; or the outer cover may be composed of an outer sheet 22 continuous from the front section A to the rear section B through the crotch section C and an inner sheet 23 disposed in either the front section A or the rear section B.

While in the foregoing embodiments both the front section A and the rear section B have the waist elasticized region G1 and the lower elasticized region G2, the present invention embraces an absorbent article having the waist elasticized region G1 and/or the lower elasticized region G2 in either one of the front section A and the rear section B.

Either one or both of the outer sheet 22 and the inner sheet 23 may optionally be folded back along the edge 51 of the waist opening 5 over the skin facing side of the inner sheet 23 to form a folded-over portion.

The present invention provides an absorbent article that is prevented from sagging after fluid absorption and exhibits good conformability to the wearer's body.

## Claims

1. An absorbent article comprising an absorbent assembly (3) having an absorbent member (33) and an outer cover (2) disposed on a non-skin facing side of the absorbent assembly (3), having a front section (A) adapted to be worn about the front of a wearer, a rear section (B) adapted to be worn about the back of the wearer, and a crotch section (C) located between the front and rear sections, and having a longitudinal direction (X) corresponding to the direction from the front section (A) to the rear section (B) through the crotch section (C) and a lateral direction (Y) perpendicular to the longitudinal direction (X),

   the outer cover (2) comprising an outer sheet (22) on a non-skin facing side defining an exterior side of the absorbent article, an inner sheet (23) on a skin facing side of the outer sheet (22), and a plurality of elastic members (24) between the outer and inner sheets,
   the outer cover (2) having a waist elasticized region (G1) longitudinally distal to the absorbent assembly (3) and a lower elasticized region (G2) located longitudinally proximal to the waist elasticized region (G1) in a region longitudinally overlapping the absorbent assembly (3),
   the outer and inner sheets (22, 23) being bonded at bonds (26) spaced apart in the lateral direction in a pattern forming between laterally adjacent bonds (26) a non-bonded region (25) where the outer and the inner sheets (22, 23) are not bonded to each other continuously in the longitudinal direction,
   the outer sheet (22) being configured to bulge away from a wearer's skin upon contraction of the elastic members (24) to form a plurality of longitudinally extending folds, and
   the waist elasticized region (G1) having a lower bond strength than the lower elasticized region (G2), the bond strength being measured in accordance with the method described in paragraph [0043] of the A1-publication.

2. The absorbent article according to claim 1, wherein the absorbent member (33) has a larger basis weight of an absorbent material in a region longitudinally overlapping the lower elasticized region (G2) than in a region longitudinally non-overlapping the lower elasticized region (G2).

3. The absorbent article according to claim 1 or 2, wherein the absorbent member (33) has a longitudinally extending fiber-free region (37) in a region longitudinally non-overlapping the lower elasticized region (G2).

4. The absorbent article according to any one of claims 1 to 3, wherein the elastic member (24) in the waist elasticized region (G1) has a smaller extensional stress than the elastic member (24) in the lower elasticized region (G2).

5. The absorbent article according to any one of claims 1 to 4, wherein the elastic member (24) in the waist elasticized region (G1) has a smaller fineness than the elastic member (24) in the lower elasticized region (G2).

6. The absorbent article according to any one of claims 1 to 5, wherein the outer cover (2) further comprises an inward sheet on the skin facing side of the inner sheet (23), at least one of the inner sheet (23) and the inward sheet having higher hydrophilicity than the outer sheet (22).

7. The absorbent article according to any one of claims 1 to 6, wherein at least one of the outer and inner sheets (22, 23) contains cellulosic fibers.

8. The absorbent article according to claim 7, wherein the outer sheet (22) contains cellulosic fibers and has a folded-over portion that is folded toward the skin facing side.

9. The absorbent article according to any one of claims 1 to 8, wherein the outer sheet (22) and the inner sheet (23) contain a softening agent at different contents.

10. The absorbent article according to any one of claims 1 to 9, wherein the inner sheet (23) has a higher coefficient of friction than the outer sheet (22).

11. The absorbent article according to any one of claims 1 to 10, wherein the inner sheet (23) has a higher fiber density than the outer sheet (22).

12. The absorbent article according to any one of claims 1 to 11, wherein the bonds (26) are fusion bonds, and the fusion bond of the waist elasticized region (G1) has a smaller cross-sectional fusion area than the fusion bond of the lower elasticized region (G2).

13. The absorbent article according to any one of claims 1 to 11, wherein the bonds (26) are hot-melt adhesive bonds, and the hot-melt adhesive bonds in the waist elasticized region (G1) have a smaller penetration depth of hot-melt adhesive than those in the lower elasticized region (G2).

14. The absorbent article according to any one of claims 1 to 13, wherein the waist elasticized region (G1) has a smaller bond area per unit area than the lower elasticized region (G2).

15. The absorbent article according to any one of claims 1 to 14, wherein the bonds (26) are continuous in the longitudinal direction.

**Patentansprüche**

1. Saugfähiger Artikel, umfassend eine saugfähige Anordnung (3) mit einem saugfähigen Element (33) und einer äußeren Hülle (2), die auf einer der Haut nicht zugewandten Seite der saugfähigen Anordnung (3) angeordnet ist, mit einem vorderen Abschnitt (A), der dazu angepasst ist, um die Vorderseite eines Trägers getragen zu werden, einem hinteren Abschnitt (B), der dazu angepasst ist, um die Hinterseite des Trägers getragen zu werden, und einem zwischen dem vorderen und dem hinteren Abschnitt befindlichen Schrittabschnitt (C) und mit einer Längsrichtung (X), die der Richtung von dem vorderen Abschnitt (A) zu dem hinteren Abschnitt (B) über den Schrittabschnitt (C) entspricht, und einer Lateralrichtung (Y) senkrecht zu der Längsrichtung (X),

wobei die äußere Hülle (2) Folgendes umfasst: eine äußere Folie (22) auf einer der Haut nicht zugewandten Seite, die eine Außenseite des saugfähigen Artikels definiert, eine innere Folie (23) auf einer der Haut zugewandten Seite der äußeren Folie (22) und eine Vielzahl von elastischen Elementen (24) zwischen der äußeren und der inneren Folie,
wobei die äußere Hülle (2) Folgendes aufweist: einen elastischen Taillenbereich (G1) in Längsrichtung distal der saugfähigen Anordnung (3) und einen unteren elastischen Bereich (G2), der sich in Längsrichtung proximal des elastischen Taillenbereichs (G1) in einem Bereich, der die saugfähige Anordnung (3) in Längsrichtung überlappt, befindet,
wobei die äußere und die innere Folie (22, 23) an Verbindungen (26) verbunden sind, die in der Lateralrichtung in einem Muster voneinander beabstandet sind, die zwischen lateral benachbarten Verbindungen (26) einen nicht verbundenen Bereich (25) bilden, wobei die äußere und die innere Folie (22, 23) in der Längsrichtung nicht durchgehend miteinander verbunden sind,
wobei die äußere Folie (22) dazu konfiguriert ist, sich, wenn sich die elastischen Elemente (24) zusammenziehen, von der Haut eines Trägers weg zu wölben, um eine Vielzahl von sich in Längsrichtung erstreckenden Falten zu bilden, und

wobei der elastische Taillenbereich (G1) eine geringere Verbindungsfestigkeit aufweist als der untere elastische Bereich (G2), wobei die Verbindungsfestigkeit gemäß dem in Absatz [0043] der A1-Veröffentlichungsschrift gemessen wird.

2. Saugfähiger Artikel nach Anspruch 1, wobei das saugfähige Element (33) in einem den unteren elastischen Bereich (G2) in Längsrichtung überlappenden Bereich ein höheres Riesgewicht eines saugfähigen Materials aufweist als in einem den unteren elastischen Bereich (G2) in Längsrichtung nicht überlappenden Bereich.

3. Saugfähiger Artikel nach Anspruch 1 oder 2, wobei das saugfähige Element (33) in einem den unteren elastischen Bereich (G2) in Längsrichtung nicht überlappenden Bereich einen sich in Längsrichtung erstreckenden faserfreien Bereich (37) aufweist.

4. Saugfähiger Artikel nach einem der Ansprüche 1 bis 3, wobei das elastische Element (24) in dem elastischen Taillenbereich (G1) eine geringere Zugspannung aufweist als das elastische Element (24) in dem unteren elastischen Bereich (G2).

5. Saugfähiger Artikel nach einem der Ansprüche 1 bis 4, wobei das elastische Element (24) in dem elastischen Taillenbereich (G1) eine geringere Feinheit aufweist als das elastische Element (24) in dem unteren elastischen Bereich (G2).

6. Saugfähiger Artikel nach einem der Ansprüche 1 bis 5, wobei die äußere Hülle (2) ferner eine inwendige Folie auf der der Haut zugewandten Seite der inneren Folie (23) umfasst, wobei mindestens eine von der inneren Folie (23) und der inwendigen Folie eine höhere Hydrophilität aufweist als die äußere Folie (22).

7. Saugfähiger Artikel nach einem der Ansprüche 1 bis 6, wobei mindestens eine von der äußeren und der inneren Folie (22, 23) Zellulosefasern enthält.

8. Saugfähiger Artikel nach Anspruch 7, wobei die äußere Folie (22) Zellulosefasern enthält und einen umgefalteten Teil aufweist, der in Richtung der der Haut zugewandten Seite gefaltet ist.

9. Saugfähiger Artikel nach einem der Ansprüche 1 bis 8, wobei die äußere Folie (22) und die innere Folie (23) einen Weichmacher in unterschiedlichen Anteilen enthalten.

10. Saugfähiger Artikel nach einem der Ansprüche 1 bis 9, wobei die innere Folie (23) einen höheren Reibungskoeffizienten aufweist als die äußere Folie (22).

11. Saugfähiger Artikel nach einem der Ansprüche 1 bis 10, wobei die innere Folie (23) eine höhere Faserdichte aufweist als die äußere Folie (22).

12. Saugfähiger Artikel nach einem der Ansprüche 1 bis 11, wobei die Verbindungen (26) Schmelzverbindungen sind und die Schmelzverbindung des elastischen Taillenbereichs (G1) eine kleinere Schmelzquerschnittsfläche aufweist als die Schmelzverbindung des unteren elastischen Bereichs (G2).

13. Saugfähiger Artikel nach einem der Ansprüche 1 bis 11, wobei die Verbindungen (26) Schmelzklebstoffverbindungen sind, und die Schmelzklebstoffverbindungen in dem elastischen Taillenbereich (G1) eine geringere Eindringtiefe von Schmelzklebstoff aufweisen als diejenigen in dem unteren elastischen Bereich (G2).

14. Saugfähiger Artikel nach einem der Ansprüche 1 bis 13, wobei der elastische Taillenbereich (G1) eine kleinere Verbindungsfläche pro Flächeneinheit aufweist als der untere elastische Bereich (G2).

15. Saugfähiger Artikel nach einem der Ansprüche 1 bis 14, wobei die Verbindungen (26) in der Längsrichtung durchgehend sind.

**Revendications**

1. Article absorbant comprenant un ensemble absorbant (3) présentant un élément absorbant (33) et une enveloppe externe (2) disposée sur un côté non orienté vers la peau de l'ensemble absorbant (3), présentant une section avant (A) adaptée pour être portée autour de l'avant d'un utilisateur, une section arrière (B) adaptée pour être portée autour de l'arrière de l'utilisateur, et une section d'entrejambe (C) située entre les sections avant et arrière, et présentant une direction longitudinale (X) correspondant à la direction de la section avant (A) à la section arrière (B) à travers la section d'entrejambe (C) et une direction latérale (Y) perpendiculaire à la direction longitudinale (X),

la couverture externe (2) comprenant une feuille externe (22) sur un côté non orienté vers la peau définissant un côté extérieur de l'article absorbant, une feuille interne (23) sur un côté orienté vers la peau de la feuille externe (22), et une pluralité d'éléments élastiques (24) entre les feuilles externe et interne,

la couverture externe (2) présentant une région élastifiée au niveau de la taille (G1) distale dans le sens longitudinal par rapport à l'ensemble absorbant (3) et une région élastifiée inférieure (G2) située à proximité dans le sens longitudinal par rapport à la région élastifiée au niveau de la taille (G1) dans une région chevauchant dans le sens longitudinal l'ensemble absorbant (3),

les feuilles externes et internes (22, 23) étant liées sur des liaisons (26) espacées dans la direction latérale selon un motif formant entre des liaisons (26) latéralement adjacentes une région non liée (25) dans laquelle les feuilles externes et internes (22, 23) ne sont pas liées l'une à l'autre de manière continue dans la direction longitudinale, la feuille externe (22) étant configurée pour se bomber à l'écart de la peau d'un utilisateur lors de la contraction des éléments élastiques (24) pour former une pluralité de plis s'étendant dans le sens longitudinal, et

la région élastifiée au niveau de la taille (G1) présentant une force de liaison inférieure à celle de la région élastifiée inférieure (G2), la force de liaison étant mesurée conformément au procédé décrit paragraphe [0043] de la publication A1.

2. Article absorbant selon la revendication 1, dans lequel l'élément absorbant (33) présente un grammage d'un matériau absorbant dans une région chevauchant dans le sens longitudinal la région élastifiée inférieure (G2) supérieur à celui dans une région ne chevauchant pas dans le sens longitudinal la région élastifiée inférieure (G2).

3. Article absorbant selon la revendication 1 ou 2, dans lequel l'élément absorbant (33) présente une région exempte de fibres (37) s'étendant dans le sens longitudinal dans une région ne chevauchant pas dans le sens longitudinal la région élastifiée inférieure (G2).

4. Article absorbant selon l'une quelconque des revendications 1 à 3, dans lequel l'élément élastique (24) dans la région élastifiée au niveau de la taille (G1) présente une contrainte d'extension inférieure à celle de l'élément élastique (24) dans la région élastifiée inférieure (G2).

5. Article absorbant selon l'une quelconque des revendications 1 à 4, dans lequel l'élément élastique (24) dans la région élastifiée au niveau de la taille (G1) présente une finesse inférieure à celle de l'élément élastique (24) dans la région élastifiée inférieure (G2).

6. Article absorbant selon l'une quelconque des revendications 1 à 5, dans lequel l'enveloppe externe (2) comprend en outre une feuille vers l'intérieur sur le côté faisant face à la peau de la feuille interne (23), au moins une de la feuille interne (23) et de la feuille vers l'intérieur présentant une hydrophilie supérieure à celle de la feuille externe (22).

7. Article absorbant selon l'une quelconque des revendications 1 à 6, dans lequel au moins une des feuilles externe et interne (22, 23) contient des fibres cellulosiques.

8. Article absorbant selon la revendication 7, dans lequel la feuille externe (22) contient des fibres cellulosiques et présente une partie repliée qui est repliée vers le côté faisant face à la peau.

9. Article absorbant selon l'une quelconque des revendications 1 à 8, dans lequel la feuille externe (22) et la feuille interne (23) contiennent un agent assouplissant à des teneurs différentes.

10. Article absorbant selon l'une quelconque des revendications 1 à 9, dans lequel la feuille interne (23) présente un coefficient de frottement supérieur à celui de la feuille externe (22).

11. Article absorbant selon l'une quelconque des revendications 1 à 10, dans lequel la feuille interne (23) présente une densité de fibres supérieure à celle de la feuille externe (22).

12. Article absorbant selon l'une quelconque des revendications 1 à 11, dans lequel les liaisons (26) sont des liaisons par fusion, et la liaison par fusion de la région élastifiée au niveau de la taille (G1) présente une surface de fusion en coupe transversale inférieure à celle de la liaison par fusion de la région élastifiée inférieure (G2).

13. Article absorbant selon l'une quelconque des revendications 1 à 11, dans lequel les liaisons (26) sont des liaisons adhésives thermofusibles, et
les liaisons adhésives thermofusibles dans la région élastifiée au niveau de la taille (G1) présentent une profondeur de pénétration d'adhésif thermofusible inférieure à celle dans la région élastifiée inférieure (G2).

14. Article absorbant selon l'une quelconque des revendications 1 à 13, dans lequel la région élastifiée au niveau de la taille (G1) présente une surface de liaison par unité de surface inférieure à celle de la région élastifiée inférieure (G2).

15. Article absorbant selon l'une quelconque des revendications 1 à 14, dans lequel les liaisons (26) sont continues dans la direction longitudinale.

Fig. 1

Fig. 2

Fig. 3

## Fig. 4(a)

## Fig. 4(b)

Fig. 5(a)

Fig. 5(b)

Fig. 6(a)

Fig. 6(b)

Fig. 7

Fig. 8

**EP 3 865 103 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008173286 A
- JP 2017121387 A

- JP 2015142721 A

**Non-patent literature cited in the description**

- Fuai Hyouka no Hyoujunka to Kaiseki (Standardization and Analysis of Hand Evaluation), Japanese Hand Evaluation and Standardization Committee (HESC). **KAWABATA** ; **HIDEO**. The Textile Machinery Soc. of Japan. 10 July 1980